(19) Europäisches Patentamt
European Patent Office
Office européen des brevets

(11) Publication number: 0 372 941 A2

(12) EUROPEAN PATENT APPLICATION

(21) Application number: 89312738.1

(22) Date of filing: 07.12.89

(51) Int. Cl.5: C07D 307/60, C07D 405/12, C07F 9/09, A61K 31/365

Claims for the following Contracting States: ES + GR.

(30) Priority: 07.12.88 US 281154

(43) Date of publication of application: 13.06.90 Bulletin 90/24

(84) Designated Contracting States: AT BE CH DE ES FR GB GR IT LI LU NL SE

(71) Applicant: ALLERGAN, INC
2525 Dupont Drive
Irvine, California 92715-1599(US)

(72) Inventor: Lee, Gary C. M.
24722 Mendocino Ct.
Laguna Hills California 92653(US)

(74) Representative: Hutchins, Michael Richard et al
Graham Watt & Co. London Road Riverhead
Sevenoaks, Kent TN13 2BN(GB)

(54) **Anti-inflammatory 5-hydroxy-2-furanones.**

(57) The invention provides compounds of the formula (I):

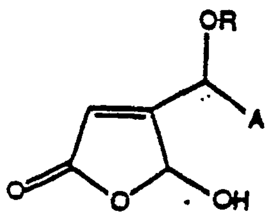

(I)

in which:
R is hydrogen, $C_1$-$C_{14}$ alkanoyl, $CONHR_3$ or $CO_2R_4$:
$R_3$ is H, phenyl or $C_1$-$C_4$alkyl;
$R_4$ is $C_1$-$C_6$ alkyl;
A is $CH_2O$-$R_1$ or

CH($C_7$-$C_{14}$ alkyl); or
|
$OCOR_2$

when R is $C_1$-$C_{14}$ alkanoyl, A may be $CH_2OCOR_2$ or $CH_2OP(O)(OR_2)R_2$;
$R_1$ is $C_7$-$C_{14}$ alkanoyl,
N-($C_6$-$C_{14}$ alkyl) carbamoyl,
naphthyl-($C_1$-$C_6$ alkylene),
pyridyl-($C_1$-$C_6$ alkylene) or

EP 0 372 941 A2

methoxyethoxymethoxymethyl; and
$R_2$ is $C_1$-$C_4$ alkyl;
pharmaceutical compositions containing them, processes for their preparation, and their use in the treatment of inflammation, allergic response and psoriasis.

# ANTI-INFLAMMATORY 5-HYDROXY-2-FURANONES

This invention relates to new 5-hydroxy-2-furanone compounds having anti-inflammatory activity, pharmaceutical compositions comprising these compounds and to methods of using them.

## Background of the Invention

Manoalide is a furanone compound isolated from marine sponge as reported by E.D. de Silva et al., Tetrahedron Letters 21:1611-1614 (1980). Anti-inflammatory, immunosuppressive and analgesic properties of manoalide are disclosed in U.S. Patent 4,447,445. Manoalide has the following structural formula:

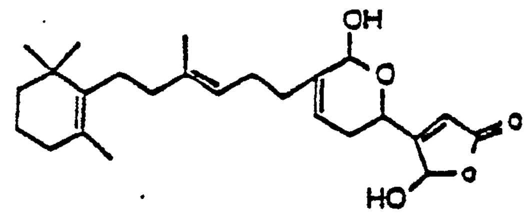

The anti-inflammatory activity of seco-manoalide and dehydro-seco-manoalide is also disclosed in U.S. Patent 4,447,445.

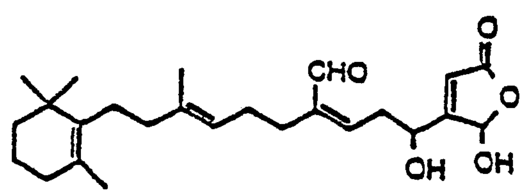

seco-manoalide

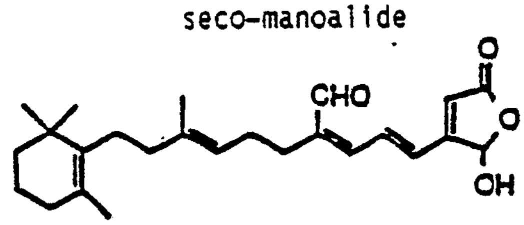

dehydro-seco-manoalide

## THE INVENTION

The compounds of the present invention are represented by the following formula:

FORMULA I

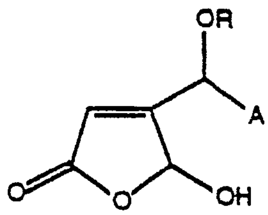

in which:
R is hydrogen, $C_1$-$C_{14}$ alkanoyl, $CONHR_3$ or $CO_2R_4$;
$R_3$ is H, phenyl or $C_1$-$C_4$ alkyl;
$R_4$ is $C_1$-$C_6$ alkyl;
A is $CH_2O$-$R_1$ or

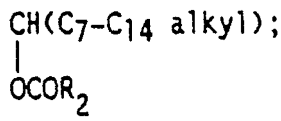

when R is $C_7$-$C_{14}$ alkanoyl, A may be $CH_2OCOR_2$ or $CH_2OP(O)(OR_2)R_2$;
$R_1$ is $C_1$-$C_{14}$ alkanoyl, N-($C_6$-$C_{14}$ alkyl) carbamoyl, naphthyl-($C_1$-$C_6$ alkylene), pyridyl-($C_1$-$C_6$alkylene) or methoxyethoxymethoxymethyl; and
$R_2$ is $C_1$-$C_4$ alkyl.

The hydroxy group in the 5-position on the furanone ring may be acylated or alkylated by standard procedures, for example, by reacting the hydroxyfuranone with an acyl anhydride or halide or with an alkyl halide to give compounds also having anti-inflammatory activity as do the 5-hydroxyfuranones.

Particular compounds of this invention are represented by Formula I in which:
R is $C_1$-$C_{14}$ alkanoyl and A is $CH_2O$-$R_1$. In these compounds, $R_1$ is, in particular, $C_8$-$C_{14}$ alkanoyl, naphthylpropyl, pyridylpropyl or methoxyethoxymethoxymethyl.

Specific compounds of this invention are:
4-[1-dodecanoyloxy-2-(2-methoxyethoxy)methoxyethyl]-5-hydroxy-2(5H)-furanone,
4-(1,2-didodecanoyloxyethyl)-5-hydroxy-2(5H)-furanone,
4-(1-acetoxy-2-[3-(2-naphthyl)propoxy]-ethyl)-5-hydroxy-2(5H)-furanone, and
4-[1-hydroxy-2-[3-(2-naphthyl)propoxy]-ethyl]-5-hydroxy-2(5H)-furanone.

Certain of the compounds of this invention contain chiral centers and accordingly, may be prepared as enantiomeric or diasteriomeric mixtures or in optically pure form. Unless otherwise specified herein, such preparations are racemates at each chiral center. However, the scope of the invention is not to be considered as limited to these forms but also to encompass the individual optical isomers of the compounds.

Compounds of the invention are prepared from 5-trimethylsilyl(TMS)-3-furaldehyde by procedures which are illustrated hereinbelow and described in more detail in the examples.

## Procedure I

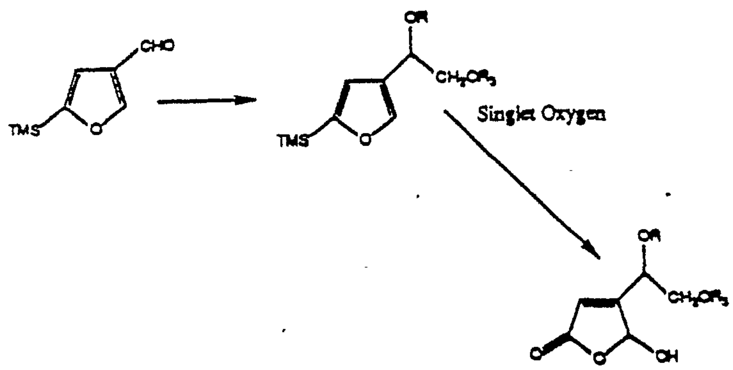


In this scheme, $R_3$ is naphthylalkylene, pyridylalkylene or methoxyethoxymethoxymethyl.

## Procedure II

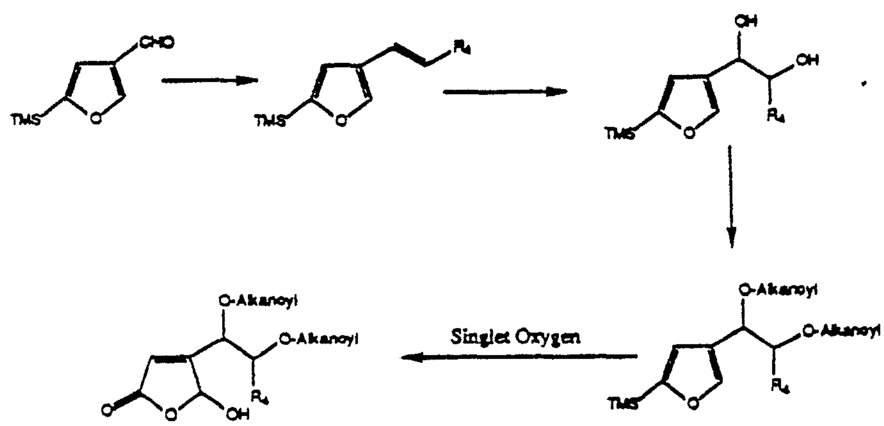


In this procedure $R_4$ is hydrogen or alkyl.

According to procedure I, the aldehyde group of 5-trimethylsilyl-3-furaldehyde is reacted with $R_3$ tributylstannylmethyl ether, the 2-OH group is optionally 0-alkanoylated and the resulting 5-trimethylsilyl-3-($CH(OR)CH_2OR_3$)-furan is converted to the corresponding 5-hydroxy-2-furanone by treating with oxygen and irradiating using an initiator such as Rose Bengal.

By procedure II, the aldehyde group of 5-trimethylsilyl-3-furaldehyde is converted to a vinyl group, for example by Wittig reaction with an alkyl triphenylphosphonium bromide/butyl lithium; the vinyl group is oxidized using, for example, osmium tetroxide; the resulting 1,2-dihydroxy groups are O-alkanoylated; and the resulting intermediate is oxidized as described above to give the 5-hydroxy-2-furanone.

The 5-trimethylsilyl-3-furaldehyde starting material may be prepared by brominating 3-furaldehyde to give 5-bromo-3-furaldehyde which is converted to the dimethylacetal, then treated with t-butyl lithium and trimethylsilyl chloride.

A preferred method for preparing 5-trimethylsilyl-3-furaldehyde is by reacting lithium morpholide with 5-bromo-3-furaldehyde to protect the aldehyde group, then reacting with t-butyl lithium and trimethylsilyl chloride to give 5-trimethylsilyl-3-furaldehyde.

An improved method for preparing 5-trimethylsilyl-3-furaldehyde consists of reacting lithium morpholide with 3-furaldehyde, followed by secondary-butyl lithium, followed by trimethylsilyl chloride. This method is

also advantageous for the preparation of 5-triethylsilyl- 3-furaldehyde using triethylsilylchloride. 5-triethylsilyl- 3-furaldehyde is useful as an intermediate in place of the trimethyl compound in methods described herein for preparing compounds of this invention.

In addition, this invention relates to pharmaceutical compositions containing the compounds of Formula I as active ingredients and to methods of using the compounds and pharmaceutical compositions of this invention to produce anti-inflammatory, immunosuppressant and anti-proliferative activity. These compounds are useful in treating inflammation, in suppressing unwanted immune responses and in retarding proliferation of cells. Uses include treatment of rheumatoid arthritis, osteoarthritis, rheumatic carditis and autoimmune diseases such as allergic diseases, bronchial asthma and myasthenia gravis and ocular and dermal inflammatory diseases. The compounds are useful in treating psoriasis, acne, atopic diseases and allergic conjunctivitis. They are also useful as adjuvant therapy associated with organ and tissue transplants.

The activity of the compounds of this invention is demonstrated by inhibition of the enzyme phospholipase $A_2$ in vitro and by reduction of inflammation in the mouse ear anti-inflammatory assay in vivo.

The compounds also inhibit phosphoinositide-specific phospholipase C. This activity has been reported for manoalide and may indicate anti-inflammatory utility. Bennett et al, Molecular Pharmacology 32:587-593 (1987).

Activity of the compounds may also be demonstrated by inhibition of ornithine decarboxylase, a rate limiting enzyme in cellular growth, which indicates use in treating psoriasis and neoplasis.

Compounds of the invention also modify calcium homeostasis. This activity is shown by effect on intracellular calcium levels in experiments using gastric glands, spleen cells, epithelial cells, $GH_3$ cells, etc. Calcium is inhibited from entering through the plasma membrane calcium channels and calcium release from intracellular stores is also blocked. Modification of calcium homeostasis is expected to have application in diseases of the nervous system involving modification of membrane lipids or transmitter release (Parkinson's, Alzheimer's), diseases of the cardiovascular system involving application of cardiac or vascular smooth muscle contractility and platelet aggregation (hypertension, cardiac infarction and atherosclerosis), diseases of the gastrointestinal tract such as ulcer disease, diarrhea, motility due to secretion of acid or $Cl^-$, diseases of the kidney involving renal handling of fluid and electrolytes (metabolic acidosis, alkalosis), and disease of abnormal growth (neoplasia, psoriasis).

The compounds of this invention have activity which is similar to that of manoalide, that is the compounds appear to be devoid of the endocrine properties of the glucocorticoids while having anti-inflammatory and immunosuppressive properties.

In the methods of this invention, the compounds of the invention are administered to mammals, including humans, in an effective amount to produce the desired activity, preferably in an amount of about 0.05 to 100 mg per day per kilogram of body weight. The amount of the compound depends upon the disease or condition being treated, the severity thereof, the route of administration and the nature of the host. The compounds may be administered topically, orally, parenterally or by other standard routes of administration.

Pharmaceutical compositions of this invention comprise compounds of Formula I and pharmaceutical carriers suitable for the route of administration. Standard methods for formulating pharmaceutical compositions of this type may be found in Remington's Pharmaceutical Sciences, Mack Publishing Company, Easton, PA.

For topical administration, the pharmaceutical composition may be in the form of a salve, cream, ointment, spray, powder or the like. Standard pharmaceutical carriers for such compositions may be used. Preferably, compositions for topical administration will contain 0.05-5% of the active ingredient.

A typical cream formulation may contain the following:

| Ingredient | Parts by Weight |
|---|---|
| Water/glycol mixture (15% or more glycol) | 50-99 |
| Fatty alcohol | 1-20 |
| Non-ionic surfactant | 0-10 |
| Mineral oil | 0-10 |
| Typical pharmaceutical adjuvants | 0-5 |
| Active ingredient | 0.05-5 |

A typical ointment formulation may contain the following:

| Ingredients | Parts by Weight |
|---|---|
| White petrolatum | 40-94 |
| Mineral Oil | 5-20 |
| Glycol solvent | 1-15 |
| Surfactant | 0-10 |
| Stabilizer | 0-10 |
| Active Ingredient | 0.05-5 |

For oral administration, suitable pharmaceutical carriers include mannitol, lactose, starch, magnesium stearate, talcum, glucose and magnesium carbonate. Oral compositions may be in the form of tablets, capsules, powders, solutions, suspensions, sustained release formulations, and the like.

A typical tablet or capsule may contain the following:

| Ingredients | Percent w/w |
|---|---|
| Lactose, spray-dried | 40-99 |
| Magnesium stearate | 1-2 |
| Cornstarch | 10-20 |
| Active ingredient | 0.001-20 |

Parenteral compositions are prepared in conventional suspension or solution forms, as emulsions or as solid forms for reconstruction. Suitable carriers are water, saline, dextrose, Hank's solution, Ringer's solution, glycerol, and the like. Parenteral administration is usually by injection which may be subcutaneous, intramuscular or intravenous.

The compounds of this invention may be combined with other known anti-inflammatory/immunosuppressive agents such as steroids or non-steroidal anti-inflammatory agents (NSAID) in the pharmaceutical compositions and methods described herein.

The following examples are intended to illustrate the invention but are not limiting. All temperatures are in degrees Centigrade. NMR data are recorded in delta ppm.

## Example 1

### 5-Trimethylsilyl-3-furaldehyde

n-Butyl lithium (a 1.6 M solution in hexane, 31.0 ml, 49.7 mmol) was added dropwise to a solution of morpholine (4.33 ml, 49.7 mmol; freshly distilled from barium oxide) in tetrahydrofuran at -78° under argon. After 15 minutes, a solution of 5-bromo-3-furaldehyde (7.5 g, 49.7 mmol) in tetrahydrofuran was added dropwise. Stirring was continued for 30 min. and n-butyl lithium (a 1.6 M solution in hexane; 46.6 ml, 74.5 mmol) was added dropwise. After 1 hour at -78°, chlorotrimethylsilane (18.9 ml, 149 mmol) was added and stirring continued while the cooling bath attained room temperature. The reaction mixture was quenched with 10% hydrochloric acid and the phases were separated. The aqueous phase was stirred, in the presence of ethyl ether (30 ml), with 10% hydrochloric acid at 0° C for 1/2 hour. The organic phases were combined, washed (brine), dried (magnesium sulfate) and evaporated down. The residue was distilled under vacuum to give the title aldehyde as a colorless oil b.p. 48-50°/0.25 torr.

## Example 2

Alternative Preparation of 5-Trimethylsilyl-3-furaldehyde

n-Butyl lithium (a 2.5 M solution in hexane; 28.8 ml, 72 mmol) was added to a solution of morpholine (6.28 ml, 72 mmol) in tetrahydrofuran (700 ml) at -78° under argon. After 20 minutes, 3-furaldehyde (7.0 g, 72 mmol) was added. After another 20 minutes, sec-butyl lithium (a 1.3 M solution in cyclohexane; 55.4 ml, 72 mmol) was added dropwise and stirring continued at -78° for 7 hours before trimethylsilylchloride (27 ml, 216 mmol) was added. Stirring continued overnight (14 hours) while the cooling bath was allowed to attain room temperature. The solution was poured into ice cold 10% (v/v) hydrochloric acid (200 ml) and after stirring at 0° for 10 minutes, the layers were separated. The aqueous phase was extracted with diethyl ether. All the organic phases were combined, dried (magnesium sulfate) and evaporated down to give a light brown oil, which was purified by flash chromatography on silica using 2% ethyl ether/hexane. Fractions with $R_f$ of about 0.30 (silica, 10% ethyl ether/hexane) on evaporation gave the title aldehyde as a light yellow oil, b.p. 48-50°/0.25 torr.

$^1$H NMR ($CDCl_3$) 0.29(5.9H), 6.98(5.1H), 8.25(5.1H) and 9.95(5.1H).

$^{13}$C NMR ($CDCl_3$) -2.0, 116.2, 121.9, 155.3, 164.1 and 114.5.

MS: Exact mass calculated for $C_8H_{12}O_2Si(M^+)$ 168.0607, found 168.0588.

EXAMPLE 3

2-Methoxyethoxymethyl tributylstannylmethyl ether

Tributyltin hydride (2.69 ml, 0.01 mol) was added dropwise to a solution of lithium diisopropylamide (a 1.5 M solution in cyclohexane; 6.7 ml, 0.01 mol) in tetrahydrofuran (20 ml) at 0° under argon. After 15 minutes, paraformaldehyde (300 mg, 0.01 mol) was added, followed by 2-methoxyethoxymethyl chloride (1.15 ml, 0.01 mol) after 3 hours. Stirring was continued at room temperature for 12 hours and the mixture was quenched with water. Extraction (ethyl ether) and evaporation of the dried (magnesium sulphate) extracts gave an oil, which was flash chromatographed on silica using 5% ethyl ether/petroleum ether. Fractions with $R_f$ of about 0.24 on evaporation afforded the desired ether as a colorless oil.

$^1$H NMR: ($CDCl_3$) 0.92 (m, 15H), 1.35 (m, 6H), 1.55 (m, 6H), 3.44 (s, 3H), 3.60 (m, 2H), 3.70 (m, 2H), 3.80 (dd, 2H), and 4.65 (s, 2H).

$^{13}$C NMR: ($CDCl_3$) 8.7, 13.5, 27.1, 28.8, 57.5, 66.3, 71.7 and 98.4.

MS m/e (% abundance): 398 $(M+NH_4)^+$, 9), 323(100), 321(74), 319(42), 294(55), 292(38), and 291(16).

4-[1-Dodecanoyloxy-2-(2-methoxyethoxy) methoxyethyl]-2-trimethylsilylfuran

n-Butyl lithium (a 1.6 M solution in hexane; 0.74 ml, 1.18 mmol) was added to a solution of 2-methoxyethoxymethyl tributylstannylmethyl ether (481.6 mg, 1.18 mmol) in tetrahydrofuran (5 ml) at -78° under argon. After 10 minutes, a solution of 5-trimethylsilyl-3-furaldehyde (198 mg, 1.18 mmol) was added, followed by lauroyl chloride (0.26 ml), 1.18 mmol) after 20 minutes. Stirring was continued at room temperature for 48 hours and the mixture was quenched with water. Extraction (ether) and evaporation of the dried (magnesium sulphate) extracts gave an oil, which was flash chromatographed on silica using 20% ethyl ether/petroleum ether. Fractions with $R_f$ of about 0.27 (30% ethyl ether/petroleum ether) on evaporation afforded the title ester as a pale yellow oil.

$^1$H NMR ($CDCl_3$): 0.26 (s, 9H), 0.89 (t, 3H, J=6.9 Hz), 1.27 (brs, 16H), 1.65 (m, 2H), 2.34 (t, 2H, J=7.7 Hz), 3.41 (s, 3H), 3.58 (m, 2H), 3.67 (m, 2H), 3.84 (m, 2H), 4.77 (s, 2H), 6.05 (dd, 1H, J=7.5 Hz, 3.8 Hz), 6.62 (s, 1H) and 7.66 (s, 1H).

MS m/e (% abundance): 488 $((M+NH_4)^+$, 26), 271 (100), 195 (42), 123 (25) and 90 (18).

4-[1-Dodecanoyloxy-2-(2-methoxyethoxy) methoxyethyl]-5-hydroxy-2(5H)-furanone

A mixture of 4-[1-dodecanoyloxy-2-(2-methoxyethoxy)methoxyethyl]-2-trimethylsilylfuran (229 mg, 0.49 mmol) and Rose Bengal (5 mg) in tetrahydrofuran (7 ml) was exposed to singlet oxygen for 2 hours at -78°. The residue, after solvent removal, was purified by preparative TLC (silica plate; developed with 80% ethyl ether/petroleum ether). The title ester was obtained as a colorless oil.

$^1H$ NMR ($CDCl_3$): 0.89 (t, 3H, J=6.9 Hz), 1.27 (brs, 16H), 1.65 (brt, 2H), 2.43 (t, 2H, J=7.8 Hz), 3.40 (s, 3H), 3.05 (m, 2H), 3.30 (m, 2H), 3.95 (m, 2H), 4.74 (s, 2H), 5.70 (brm, 1H), 6.11 (s, 1H), and 6.14 (s, 1H).

$^{13}C$ NMR ($CDCl_3$): 14.1, 22.7, 24.8, 29.1, 29.2, 29.3, 29.4, 29.6, 31.9, 34.0, 58.9, 67.2, 67.4, 68.5, 71.7, 95.6, 97.9, 98.1, 120.2, 169.6 and 172.9.

MS m/e exact mass calculated for $C_{22}H_{42}NO_8(M+NH_4)^+$ 448.2910, found 448.2890.

EXAMPLE 4

2-Trimethylsilyl-4-vinylfuran

n-Butyl lithium (a 1.6 M solution in hexane; 2.23 ml, 3.57 mmol) was added dropwise to a suspension of methyltriphenylphosphonium bromide (262 mg, 0.73 mmol) in tetrahydrofuran (8 ml) at 0° under argon. After 20 minutes, a solution of 5-trimethylsilyl-3-furaldehyde (102 mg, 0.61 mmol) in tetrahydrofuran (1/2 ml) was added. Stirring was continued for 18 hours while the cooling bath attained room temperature. The mixture was quenched with methanol/water (1:1, 20 ml) and extracted with pentane. Evaporation of the dried (magnesium sulphate) extracts gave an oil, which was flash chromatographed on silica using pentane. The title vinylfuran was obtained as a colorless oil.

$^1H$ NMR ($CDCl_3$): 0.31 (s, 9H), 5.15 (d, 1H, J=10.2 Hz), 5.50 (d, 1H, J=17.6 Hz), 6.64 (dd, 1H, J=17.6 Hz, 10.2 Hz), 6.82 (s, 1H) and 7.65 (s, 1H).

MS m/e (% abundance): 184 (($M+NH_4^+$), 22), 158 (14), 108 (26), 90 (23), 74 (68) and 60 (100).

4-(1,2-Dihydroxyethyl)-2-trimethylsilylfuran

2-Trimethylsilyl-4-vinylfuran (272 mg, 1.64 mmol) was added to a mixture of 4-methylmorpholine N-oxide (203 mg, 1.74 mmol), osmium tetroxide (a 2.5% by weight solution in tert-butanol; 0.1 ml), water (3.5 ml) and acetone (1.5 ml) at room temperature under argon. Stirring was continued for 19 hours and most of the acetone was evaporated under vacuum. Sodium bisulphite was added to the residue and the pH of the solution was adjusted to 1 with dilute sulphuric acid. After being saturated with sodium chloride, the solution was extracted thoroughly with ethyl acetate. Evaporation of the dried (magnesium sulphate) extracts afforded an oil, which was purified by preparative TLC (silica plate; developed with 60% ethyl ether/petroleum ether). The title diol was obtained as a pale yellow oil.

$^1H$ NMR (mixture of diasteriomers) ($CDCl_3$): 0.29 (s, 9H), 0.35 (s, 9H), 2.2 (br, 2H), 3.70-3.85 (m, 2H), 4.85 (dd, 1H, J=3.9 Hz, 7.3 Hz), 4.95 (m, 2H), 6.50 (d, 1H), 6.65 (s, 1H), 7.60 (d, 1H) and 7.66 (s, 1H).

$^{13}C$ NMR ($CDCl_3$): -1.8, -1.0, 66.9, 67.3, 67.7, 108.4, 118.3, 124.3, 134.2, 143.7, 146.5, 156.2 and 161.5.

MS m/e (% abundance): 200 ($M^+$, 18), 169 (100), 153 (22), 139 (9) and 73 (73).

4-(1,2-Didodecanoyloxyethyl)-2-trimethylsilylfuran

tert-Butyl lithium (a 1.7 M solution in pentane; 0.34 ml, 0.57 mmol) was added dropwise to a solution of 4-(1,2-dihydroxyethyl)-2-trimethylsilylfuran (52 mg, 0.26 mmol) in tetrahydrofuran (1 ml) at 0° under argon. After 5 minutes, lauroyl chloride (0.13 ml, 0.57 mmol) was added and stirring was continued at room temperature for 15 hours. The mixture was quenched with water and extracted with ether. Evaporation of the dried (magnesium sulphate) extracts gave an oil, which was purified by preparative TLC (silica plate;

developed with 5% ethyl ether/petroleum ether). The title diester was obtained as a pale yellow oil.

$^1H$ NMR ($CDCl_3$): 0.28 (s, 9H), 0.91 (t, 6H, J=6.9 Hz), 1.29 (brs, 32H), 1.60 (m, 4H), 2.35 (m, 4H), 4.35 (m, 2H), 6.12 (m, 1H), 6.69 (s, 1H) and 7.67 (s, 1H).

### 4-(1,2-Didodecanoyloxyethyl)-5-hydroxy-2(5H)-furanone

A mixture of 4-(1,2-didodecanoyloxyethyl)-2-trimethylsilylfuran (97.1 mg, 0.17 mmol) and Rose Bengal (5 mg) in tetrahydrofuran (8 ml) was exposed to singlet oxygen for 2-1/2 hours at -78°. The residue, after solvent removal, was purified by preparative TLC (20x20 cm, 500μ silica plate; developed with 60% ethyl ether/petroleum ether). The title diester was obtained as a colorless waxy solid.

$^1H$ NMR ($CDCl_3$) 0.89 (t, 6H, J=6.9 Hz), 1.27 (brs, 16H), 1.65 (m, 4H), 2.34 (t, 2H, J=7.6 Hz), 2.41 (t, 2H, J=8.0 Hz), 4.35-4.55 (m, 2H), 5.75 (t, 1H), 6.09 (s, 1H) and 6.15 (s, 1H).

$^{13}C$ NMR ($CDCl_3$): 14.1, 22.7, 24.8, 28.1, 28.3, 28.5, 28.6, 28.7, 29.1, 29.4, 29.5, 29.6, 31.9, 34.0, 62.7, 67.6, 97.7, 121.9, 161.5, 168.9 and 172.5.

MS m/e exact mass calculated for $C_{30}H_{56}NO_7$ $(M+NH_4)^+$ 542.4057, found 542.4054.

## EXAMPLE 5

### Methyl 3-(2-naphthyl)propen-2-oate

A mixture of 2-naphthaldehyde (4.86 g, 31.1 mmol) and methyl (triphenylphosphoranylidene)acetate (11.45 g, 34.2 mmol) in tetrahydrofuran (50 ml) was stirred at room temperature for 2 days. The residue, after solvent removal, was dissolved in methanol/water (100 ml, 1:1) and extracted with ethyl ether/petroleum ether (7:3). Evaporation of the dried (magnesium sulphate) extracts gave an oil, which was flash chromatographed on silica using 10% ethyl ether/petroleum ether. Fractions with $R_f$ of about 0.18 on evaporation afforded the title ester as colorless shiny plates: mp 93-4°.

$^1H$ NMR ($CDCl_3$): 3.85 (s, 3H), 6.56 (d, 1H, J=16 Hz) and 7.50-8.00 (m, 8H).

MS m/e (% abundance): 212 ($M^+$, 100), 197 (3), 181 (76), 152 (57), 127 (10) and 76 (20).

### 3-(2-Naphthyl)propan-1-ol

A solution of methyl 3-(2-naphthyl)propen-2-oate (3.22 g, 15.2 mmol) in tetrahydrofuran (15 ml) was added dropwise to a suspension of lithium aluminium hydride (1.73 g, 45.5 mmol) in refluxing tetrahydrofuran (100 ml). After 15 hours reflux, the mixture was cooled and quenched with excess ethyl acetate. Extraction (ethyl ether) and evaporation of the dried (magnesium sulphate) extracts gave an oil, which was flash chromatographed on silica using 40% ethyl ether/petroleum ether. Fraction with $R_f$ of about 0.08 (30% ethyl ether/petroleum ether) on evaporation afforded the title alcohol as a pale yellow viscous oil.

$^1H$ NMR ($CDCl_3$): 2.03 (p, 2H, J=7.9 Hz), 2.93 (t, 2H, J=7.3 Hz), 3.76 (t, 2H, J=6.5 Hz) and 7.35-7.90 (m, 7H).

MS m/e (% abundance): 186 ($M^+$, 49), 167 (11), 142 (100), 141 (58), 128 (12) and 115 (21).

### (2-Naphthyl)propyl tributylstannylmethyl ether

Potassium hydride (315 mg, 7.87 mmol) was added to a solution of 3-(2-naphthyl)propan-1-ol (1.33 g, 7.16 mmol) in tetrahydrofuran (10 ml) at room temperature under argon. After 1/2 hour, a solution of tributylstannylmethyliodide (3.09 g, 7.16 mmol) in tetrahydrofuran (2 ml) was added. After stirring at room temperature for 10 days, the mixture was diluted with hexane and washed thoroughly with water. Evaporation of the dried (magnesium sulphate) organic layer gave an oil, which was flash chromatographed

on silica using 5% ethyl ether/petroleum ether. The title ether was obtained as a colorless oil.

$^1$H NMR ($CDCl_3$): 0.90 (m, 15H), 1.35 (m, 6H), 1.55 (m, 6H), 1.96 (p, 2H, J=7.9 Hz), 2.85 (t, 2H, J=8.1 Hz), 3.37 (t, 2H, J=5.8 Hz), 3.75 (dd, 2H) and 7.30-7.90 (m, 7H).

$^{13}$C NMR ($CDCl_3$): 9.0, 13.8, 27.3, 29.0, 29.2, 29.3, 31.3, 32.5, 61.8, 74.4, 77.4, 125.0, 125.8, 126.4, 127.4, 127.5, 127.8, 132.0, 133.6 and 139.7.

MS m/e exact mass calculated for $C_{26}H_{42}OSn$ ($M^+$) 490.2258, found 490.250

## 4-[1-Acetoxy-2-[3-(2-naphthyl)-propoxy]ethyl]-2-trimethylsilylfuran

n-Butyl lithium (a 1.6 M solution in hexane; 0.69 ml, 1.1 mmol) was added dropwise to a solution of (2-naphthyl)propyl tributylstannylmethyl ether (511.9 mg, 1.05 mmol) in tetrahydrofuran (5 ml) at -78° under argon. After 5 minutes, a solution of 5-trimethylsilyl-3-furaldehyde (176 mg, 1.05 mmol) in tetrahydrofuran (1/2 ml) was added, followed by acetic anhydride (0.1 ml, 1.58 mmol) after 25 minutes. Stirring was continued at -78° for 1 hour and the mixture was quenched with water. Extraction (ethyl ether) and evaporation of the dried (magnesium sulphate) extracts gave an oil, which was flash chromatographed using 10% ethyl ether/petroleum ether. The title ester was obtained as a pale yellow oil.

$^1$H NMR ($CDCl_3$): 0.25 (s, 9H), 2.00 (m, 2H), 2.08 (s, 3H), 2.09 (s, 3H), 2.84 (m, 2H), 3.40-3.80 (m, 4H), 5.29 (s, 1H), 6.00 (m, 1H), 6.63 (s, 1H) and 7.30-7.85 (m, 8H).

MS m/e (% abundance): 410 ($M^+$, 5), 350 (21), 170 (14), 169 (100), 141 (53) and 73 (100).

## 4-[1-Acetoxy-2-[3-(2-naphthyl)-propoxy]ethyl]-5-hydroxy-2(5H)-furanone

A mixture of 4-[1-acetoxy-2-[3-(2-naphthyl) propoxyl]-2- trimethylsilylfuran (230 mg, 0.56 mmol) and Rose Bengal (5 mg) in tetrahydrofuran (7 ml) was exposed to singlet oxygen at -78°C for 2 hours. The residue, after solvent removal, was purified by preparative TLC (silica plate developed with 60% ethyl ether/petroleum ether). The title furanone was obtained as a pale yellow oil.

$^1$H NMR ($CDCl_3$): 2.02 (p, 2H, J=7.9 Hz), 2.17 (s, 3H), 2.85 (t, 2H, J=7.5 Hz), 3.55 (m, 2H), 3.73 (dd, 1H, J=10.9 Hz, 4.9 Hz), 3.80 (m, 1H), 4.90 (br, 1H), 5.68 (br, 1H), 6.12 (br, 2H) and 7.30-7.90 (m, 7H).

$^{13}$C NMR (mixture of diasteromers) ($CDCl_3$): 18.0, 20.7, 20.8, 30.6, 30.8, 32.1, 58.4, 67.9, 68.7, 69.8, 70.5, 70.7, 70.9, 97.3, 98.1, 119.7, 125.1, 125.9, 126.0, 126.2, 126.4, 126.5, 126.8, 127.1, 127.3, 127.5, 127.8, 127.9, 132.0, 133.5, 138.8, 139.1, 146.9, 170.0 and 170.3.

MS m/e exact mass calculated for $C_{21}H_{22}O_6$ ($M^+$) 370.1416, found 370.1426.

## EXAMPLE 6

## 4-[1-Hydroxy-2-[3-(2-naphthyl)-propoxy]ethyl]-2-trimethylsilylfuran

n-Butyl lithium (a 1.6 M solution in hexane; 0.56 ml, 0.94 mmol) was added dropwise to a solution of (2-naphthyl)propyl tributylstannylmethyl ether (437 mg, 0.89 mmol), prepared as in Example 3, in tetrahydrofuran (5 ml) at -78° under argon. After 5 minutes, a solution of 5-trimethylsilyl-3-furaldehyde (150 mg, 0.89 mmol) in tetrahydrofuran (1 ml) was added. Stirring was continued at -78° for 2 hours and at room temperature for 1 hour. The mixture was quenched with water and extracted with ethyl ether. Evaporation of the dried (magnesium sulphate) extracts gave an oil, which was flash chromatographed on silica using 30% ethyl ether/hexane. The title alcohol was obtained as a colorless oil.

$^1$H NMR ($CDCl_3$): 0.26 (s, 9H), 2.05 (p, 2H, J=7.5 Hz), 2.89 (t, 2H, J=7.5 Hz), 3.55 (m, 4H), 4.85 (m, 1H), 6.65 (s, 1H), 7.30-7.50 (m, 4H), 7.64 (s, 1H) and 7.75-7.90 (m, 2H).

MS m/e (% abundance): 368 ($M^+$, 23), 350 (9), 169 (100), 141 (55), 115 (15) and 73 (52).

## 4-[1-Hydroxy-2-[3-(2-naphthyl)-propoxy]ethyl]-5-hydroxy-2(5H)-furanone

A mixture of 4-[1-hydroxy-2-[3-(2-naphthyl) propoxy]ethyl]-2- trimethylsilylfuran (160 mg, 0.44 mmol) and Rose Bengal (5 mg) in tetrahydrofuran (6 ml) was exposed to singlet oxygen at -78° for 1-1/2 hours. The residue, after solvent removal, was purified by preparative TLC (silica plate developed with 80% ethyl ether/hexane). The title furanone was obtained as a low-melting off-white solid.

$^1H$ NMR ($CDCl_3$): 1.97 (p, 2H, J = 7.2 Hz), 2.82 (t, 2H, J = 7.4 Hz), 3.50 (m, 4H), 4.73 (br, 1H), 6.09 (s, 1H), 6.14 (s, 1H) and 7.30-7.90 (m, 7H).

$^{13}C$ NMR ($CDCl_3$): 30.7, 32.3, 67.4, 71.0, 72.7, 98.0, 119.1, 125.3, 126.0, 126.4, 127.1, 127.4, 127.5, 127.6, 128.0, 132.0, 133.5, 138.9 and 170.8.

MS m/e exact mass calculated for $C_{19}H_{20}O_5$ ($M^+$) 328.1310, found 328.1291.

EXAMPLE 7

3-(2-Pyridyl)-2-propyn-1-ol

A mixture of 2-bromopyridine (5 g, 31.6 mmol), palladium (II) acetate (5 mg), copper (I) iodide (5 mg), triphenylphosphine (5 mg) in triethylamine (50 ml) was deaerated with argon for 5 minutes. After propagyl alcohol (2.2 ml, 38.0 mmol) was added, the solution was rapidly heated to reflux under argon and conditions maintained for 17 h. On cooling, the mixture was poured into 2M sodium hydroxide (ca. 30 ml) and extracted thoroughly with ethyl acetate. Evaporation of the dried (magnesium sulphate) extracts gave an oil, which was flash chromatographed on silica using 80% ethyl acetate/chloroform. Fractions with $R_f$ of about 0.23 on evaporation gave the title alcohol as a light tan solid: mp 78-80°.

$^1H$ NMR ($CDCl_3$): 3.42 (br, 1H), 4.59 (s, 2H), 7.30 (m, 1H), 7.48 (d, 1H, J = 7.9 Hz), 7.71 (dt, 1H, J = 7.6 Hz, 1.9 Hz) and 8.60 (d, 1H, J = 4.8 Hz).

$^{13}C$ NMR ($CDCl_3$): 50.5, 83.4, 89.2, 122.9, 127.1, 136.5, 142.5 and 149.3.

MS m/e (% abundance): 133 ($M^+$, 25), 104 (100) and 78 (29).

3-(2-Pyridyl)-1-propanol

A solution of 3-(2-pyridyl)-2-propyn-1-ol (540 mg, 4.06 mmol) in ethyl acetate (15 ml) and ethanol (3 ml) was hydrogenated over 10% palladium on carbon at room temperature for 13 h. The mixture was filtered through celite and the filtrate on evaporation gave an oil, which was flash chromatographed on silica using 3% methanol/dichloromethane. Fractions with $R_f$ of about 0.09 on evaporation gave the title alcohol as a yellow oil.

$^1H$ NMR ($CDCl_3$): 2.02 (p, 2H, J = 7.2 Hz), 3.01 (t, 2H, J = 7.0 Hz), 3.75 (t, 2H, J = 6.8 Hz), 4.3 (br, 1H), 7.15 (m, 2H), 7.65 (dt, 1H, J = 7.6 Hz, 1.9 Hz) and 8.52 (d, 1H, J = 4.8 Hz).

MS m/e (% abundance): 138 ($M^+$ + 1, 100), 120 (21) and 93 (18).

3-(2-Pyridyl)propyl tributylstannylmethyl ether

Potassium hydride (52 mg, 1.30 mmol) was added to a solution of 3-(2-pyridyl)propan-1-ol (161.8 mg, 1.18 mmol) in tetrahydrofuran (3 ml) at room temperature under argon. After 30 minutes, a solution of tributylstannylmethyl iodide (510 mg, 1.18 mmol) in tetrahydrofuran (0.5 ml) was added. After stirring was continued at room temperature for 9 days, the mixture was diluted with ether and washed thoroughly with water. Evaporation of the dried (magnesium sulphate) organic layer gave an oil, which was flash chromatographed on silica using 60% ethyl ether/hexane. Fractions with $R_f$ of about 0.53 on evaporation gave the title ether as a bright yellow oil.

$^1H$ NMR ($CDCl_3$): 0.95 (m, 15H), 1.35 (m, 6H), 1.55 (m, 6H), 2.0 (p, 2H, J = 6.3 Hz), 2.86 (t, 2H, J = 7.8 Hz), 3.38 (t, 2H, J = 6.3 Hz), 3.74 (t, 2H), 7.15 (m, 2H), 7.60 (dt, 1H, J = 7.5 Hz, 1.8 Hz) and 7.55 (bd, 1H).

$^{13}C$ NMR ($CDCl_3$): 8.7, 13.5, 27.1, 28.9, 29.4, 34.7, 61.6, 74.4, 120.6, 122.6, 135.8, 148.9 and 161.7.

MS m/e (% abundance): 441 ($M^+$, 1), 384 (47), 383 (18), 382 (35), 381 (14), 380 (20), 354 (18), 338 (30), 235 (19), 211 (10), 179 (29), 177 (27), 175 (17), 150 (11), 121 (22), 120 (100) and 91 (57).

4-[1-Acetoxy-2-[3-(2-pyridyl)-propoxy]ethyl]-2-trimethylsilylfuran

n-Butyl lithium (a 1.6 M solution in hexane: 0.46 ml, 0.74 mmol) was added dropwise to a solution of (2-pyridyl)propyl tributylstannyl ether (309.2 mg, 0.70 mmol) in tetrahydrofuran (5 ml) at -78° under argon. After 5 minutes, a solution of 5-trimethylsilyl-3-furaldehyde (118 mg, 0.70 mmol) in tetrahydrofuran (1.5 ml) was added, followed by acetic anhydride (0.1 ml, 1.05 mmol) after 1 hour. Stirring was continued at -78° for 4 hours and the mixture was quenched with water. Extraction (ethyl ether) and evaporation of the dried (magnesium sulphate) extracts gave an oil, which was flash chromatographed on silica using 40% ethyl acetate/hexane. Fractions with $R_f$ of about 0.12 on evaporation afforded the title ester as a yellow oil.

$^1H$ NMR ($CDCl_3$): 0.28 (s, 9H), 2.08 (p, 2H, J=8.1 Hz), 2.12 (s, 3H), 2.89 (t, 2H, J=7.8 Hz), 3.55 (m, 2H), 3.65 (dd, 1H, J=6.6 Hz, 0.9 Hz), 3.77 (dd, 1H, J=10.8 Hz, 3.4 Hz), 6.02 (m, 1H), 6.65 (s, 1H), 7.15 (m, 2H), 7.63 (dt, 1H, J=7.7 Hz, 1.8 Hz), 7.69 (s, 1H) and 8.56 (M, 1H).

MS m/e (% abundance): 362 ($M^+$+1, 1) 346 (5), 318 (1), 224 (26), 182 (32), 169 (26), 150 (11), 120 (100), 93 (70) and 92 (11).

4-[1-Acetoxy-2-[3-(2-pyridyl)-propoxy]ethyl]-5-hydroxy-2(5H)-furanone

A mixture of 4-[1-acetoxy-2-[3-(2-pyridyl)-propoxy]ethyl]-2-trimethylsilylfuran (120 mg, 0.33 mmol) and Rose Bengal (5 mg) in tetrahydrofuran (6 ml) was exposed to singlet oxygen at -78° for 2 h. The residue, after solvent removal, was purified by preparative TLC (silica plate developed with 80% ethyl acetate/hexane). The title furanone was obtained as a colorless oil.

$^1H$ NMR (mixture of diasteriomers) ($CDCl_3$): 2.0 (brm, 2H), 2.20 (s, 3H), 2.92 (m, 2H), 3.55 (brm, 2H), 3.85 (brm, 2H), 5.85 (br, 1H), 6.11 (brs, 1H), 6.37 (brs, 1H), 7.25 (m, 2H), 7.72 (t, 1H, J=7.6 Hz) and 8.48 (d, 1H, J=4.5 Hz).

$^{13}C$ NMR ($CDCl_3$): 20.8, 29.9, 33.7, 68.0-69.0 (br), 98.3 (br), 119.1 (br), 121.7, 123.5, 137.6, 141.8, 160.8, 160.9, 165.2, 169.7 and 170.1.

MS: Exact mass calculated for $C_{17}H_{20}NO_5(M - H)^+$: 318.1341, found 318.1399.

## EXAMPLE 8

3-[1-Hydroxy-2-(2-methoxyethoxy)methoxyethyl]-5-trimethylsilylfuran

A solution of 2-methoxyethoxymethyl tributylstannylmethyl ether (620 mg, 1.5 mmole) in tetrahydrofuran (THF) (5 ml) at -78°C was treated with n-butyl lithium (92.5M solution in hexane: 0.67 ml, 1.67 mmole). After 10 min., a solution of 5-trimethylsilyl-3-furaldehyde (254 mg, 1.5 mmole) in THF (2 ml) was added. The solution was stirred for 3 hours while the cooling bath warmed to room temperature. The mixture was quenched with water and the aqueous phase extracted with ethyl ether. The combined organic portions were dried over $MgSO_4$, filtered and concentrated. Purification by flash chromatography (silica, 50% ethyl ether/hexane) gave 216.5 mg of the title compound as a light yellow oil.

$^1H$ NMR ($CDCl_3$): 7.62 (s, 1H), 6.62 (s, 1H), 4.87 (m, 1H), 4.80 (s, 2H), 3.75 (m, 4H), 3.55 (m, 2H), 3.4 (s, 3H), 0.24 (s, 9H).

$^{13}C$ NMR ($CDCl_3$): 160.9, 143.5, 124.9, 118.3, 95.8, 73.4, 71.5, 70.0, 65.8, 58.7, 1.8.

MS m/e exact mass calculated for $C_{13}H_{24}O_5Si$ 288.1393 ($M^+$), found 288.1392.

4-[1-Hydroxy-2-(2-methoxyethoxy)methoxyethyl]-5-hydroxy-2(5H)-furanone

A mixture of 3-[1-hydroxy-2-(2-methoxyethoxy) methoxyethyl]-5-trimethylsilylfuran (135 mg, 0.59 mmole) and Rose Bengal (5 mg) in acetone (30 ml) was exposed to singlet oxygen at -78° C for 2 hours. The residue, after solvent removal, was purified by preparative TLC (silica plate: developed with 7% methanol/chloroform). The title furanone was obtained as a clear oil.

'H NMR ($CDCl_3$): 6.21 (s, 1H), 6.18 (s, 1H), 4.76 (s+t, 3H), 3.9 (d, J = 4.3 Hz, 2H), 3.76 (m, 2H), 3.61 (t, J = 4.5 Hz, 2H), 3.43 (s, 3H).

$^{13}C$ NMR ($CDCl_3$): 171, 119.1, 98.2, 96.0, 71.5, 70.9, 67.5, 67.3, 58.8.

MS m/e exact mass calculated for $C_{10}H_{20}O_7N$ 266.1239 $(M + NH_4)^+$ found 266.1241.

## EXAMPLE 9

### 3-[1-Acetoxy-2-(2-methoxyethoxy)methoxyethyl]-5-trimethylsilylfuran

A solution of 2-methoxyethoxymethyl tributylstannylmethyl ether (620 mg, 1.5 mmole) in tetrahydrofuran (THF) (5 ml) at -78° C was treated with n-butyl lithium (a 2.5 M solution in hexane: 0.67 ml, 1.67 mmole). After 10 min., a solution of 5-trimethylsilyl-3-furaldehyde (254 mg, 1.5 mmole) in THF (2 ml) was added. The solution was stirred for 3 hours while the cooling bath warmed to room temperature. Acetic anhydride (309 mg, 3.03 mmole) was added and the solution stirred at room temperature overnight. The mixture was quenched with water and the aqueous phase extracted with ethyl ether. The combined organic portions were dried over $MgSO_4$, filtered and concentrated. Purification by flash chromatography (silica, 50% ethyl ether/hexane) gave the title compound as a light yellow oil.

'H NMR ($CDCl_3$): 7.70 (s, 1H), 6.65 (s, 1H), 6.04 (m, 1H), 4.80 (s, 2H), 3.9 (m, 2H), 3.72 (m, 2H), 3.59 (m, 2H), 3.44 (s, 3H), 0.29 (s, 9H).

$^{13}C$ NMR ($CDCl_3$): 170.3, 161.3, 144.9, 121.7, 118.7, 95.4, 71.6, 70.0, 67.3, 66.9, 58.9, 21.2, -1.8.

MS m/e exact mass calculated for $C_{15}H_{26}O_6Si$ 330.1498 ($M^+$), found 330.1486.

### 4-[1-Acetoxy-2-(2-methoxyethoxy)methoxyethyl]-5-hydroxy-2(5H)-furanone

A mixture of 3-[1-acetoxy-2-(2-methoxyethoxy)methoxyethyl]-5-trimethylsilylfuran (92.6 mg, 0.28 mmole) and Rose Bengal (5 mg) in acetone (20 ml) was exposed to singlet oxygen at -78° C for 2 hours. The residue, after solvent removal, was purified by preparative TLC (silica plate: developed with ethyl acetate). The title furanone was obtained as a light yellow oil.

'H NMR ($CDCl_3$): 6.15 (s, 1H), 6.12 (s, 1H), 5.7 (br, 1H), 4.73 (s, 2H), 3.92 (m, 2H), 3.65 (m, 2H), 3.05 (m, 2H), 3.39 (s, 3H), 2.16 (s, 3H).

$^{13}C$ NMR ($CDCl_3$): 170.0, 163.8, 120.3, 98.0 97.7, 71.5, 68.7, 67.3, 67.0, 66.8, 66.7, 65.8, 58.9, 20.7.

MS exact mass calculated for $C_{12}H_{22}O_8N$ 308.1345 $(M + NH_4)^+$, found 308.1360.

## EXAMPLE 10

### (E),(Z)-3-(1-Tridecenyl)-5-trimethylsilylfuran

n-Butyl lithium (a 1.6 M solution in hexane: 5.58 ml, 8.9 mmol) was added dropwise to a solution of dodecyltriphenylphosphonium bromide (4.57 g, 8.9 mmol) in tetrahydrofuran (35 ml) at 0° under argon. After 25 min., a solution of 5-trimethylsilyl-3-furaldehyde (1 g, 5.9 mmol) in tetrahydrofuran (3 ml) was added. Stirring was continued for 1 hour at 0° and the mixture was quenched with methanol/water (1:1, 60 ml). Extraction (hexane/ether, 1:1), washing (brine) and evaporation of the dried (magnesium sulphate)

extracts gave an oil, which was purified by flash chromatography (silica) using 5% ethyl ether/hexane. Fractions with $R_f$ of about 0.8 on evaporation afforded the title olefin as a pale yellow oil.

'H NMR ($CDCl_3$) (E)-isomer: 0.31 (s, 9H), 0.92 (t, 3H, J = 7.1 Hz), 1.30 (brs, 18H), 2.29 (q, 2H, J = 7.3 Hz), 5.95, 6.00 (dt, 1H, J = 15.0 Hz, 7.5 Hz), 6.25 (d, 1H, J = 15 Hz), 6.68 (s, 1H) and 7.66 (s, 1H).

(Z)-isomer: 0.30 (s, 9H), 0.92 (t, 3H, J = 1 Hz), 1.30 (brs, 18H), 2.15 (q, 2H, J = 7.3 Hz), 5.55, 5.60 (dt, 1H, J = 11.5 Hz, 5.5 Hz), 6.15 (d, 1H, J = 11.5 Hz), 6.77 (s, 1H) and 7.58 (s, 1H). ((E):(Z) = 1:2)

MS m/e (% abundance): 320 ($M^+$, 38), 180 (25), 154 (50), 75 (15) and 73 (100).

## 3-(1,2-Dihydroxytridecyl)-5-trimethylsilylfuran

Pyridine (31 µl, 0.38 mmol), followed by osmium tetroxide (a 2.5% by weight solution in tert-butanol; 5 drops) was added to a solution of (E,(Z)-3-(tridec-1-enyl)-5-trimethylsilylfuran (111 mg, 0.35 mmol) and N-methylmorpholine N-oxide (45 mg, 0.38 mmol) and acetone (2 ml) at room temperature. After stirring for 4 days, the mixture was acidified (dilute hydrochloric acid) and extracted thoroughly with ethyl acetate. The extracts were combined, dried (magnesium sulphate) and evaporated down to give an oil, which was flash chromatographed on silica using 60% ethyl ether/hexane. Fractions with $R_f$ of about 0.26 and 0.21 (mixture of diasteriomers) on evaporation gave the title diol as an off-white solid.

'H NMR ($CDCl_3$) (mixture of diasteriomers): 0.26 (s, 3H), 0.88 (t, 3H, J = 6.5 Hz), 1.26-1.60 (m, 2H), 3.55-3.85 (ml 1H), 4.45 (d, 1H), 4.65 (d, 1H), 6.60 (s, 1H), 6.66 (s, 1H) and 7.63 (s, 1H).

MS m/e (% abundance): 354 ($M^+$, 4), 339 (2), 321 (1), 171 (17), 170 (100), 169 (87), 153 (13), 98 (16), 75 (13), 73 (59), 57 (11) and 55 (12).

## 3-(1,2-Diacetoxytridecyl)-5-trimethylsilylfuran

A mixture of 4-(1,2-dihydroxytridecyl-5-trimethylsilylfuran (110 mg, 0.31 mmol), acetic anhydride (1.5 ml) and pyridine (1.5 ml) was stirred at room temperature for 13 hours. After most of the solvent was removed under high vacuum, the residue was dissolved in dichloromethane and washed thoroughly with aqueous copper sulphate. Evaporation of the dried (magnesium sulphate) organic phase gave an oil, which was purified by flash chromatography using 10% ethyl ether/hexane. Fractions with $R_f$ of about 0.52 and 0.46 (mixture of diasteriomers) on evaporation gave the title diacetate.

'H NMR ($CDCl_3$) (mixture of diasteriomers): 0.29, 0.30 (2S, 9H), 0.92 (t, 3H, J = 7.0 Hz), 1.30 (br, 18H), 1.50 (m, 2H), 2.09, 2.10, 2.11, 2.12 (4S), 5.25 (m, 1H), 5.88 (d, 1H, J = 7.5 Hz), 5.95 (d, 1H, J = 3.5 Hz), 6.63 (s, 1H), 6.65 (s, 1H), 7.66 (s, 1H) and 7.67 (s, 1H).

MS m/e (% abundance): 438($M^+$ + 2), 423(2), 407(2), 396(1), 379(100), 337(16), 336(23), 169(23), 117-(13), 73(16) and 61(14).

## 4-(1,2-Diacetoxytridecyl)-5-hydroxy-2(5H)-furanone

A mixture of 3-(1,2-diacetoxytridecyl)-5-trimethylsilylfuran (82 mg, 0.19 mmol) and Rose Bengal (5 mg) in tetrahydrofuran (7 ml) was exposed to singlet oxygen at -78° for 3 hours. The residue, after solvent removal, was purified by preparative TLC (silica plate developed with 60% ethyl ether/hexane). The title furanone was obtained as a pale yellow oil.

'H NMR ($CDCl_3$) (mixture of diasteriomers): 0.9 (t, 3H, J = 6.4 Hz), 1.27 (brs, 18H), 1.65 (br, 2H), 2.10, 2.11, 2.17, 2.21 (4S, 12H), 5.21 (brt, 1H), 5.50-5.80 (2 brm, 2H), 6.03 (brs, 1H), 6.13 (brs, 1H) and 6.16 (brs, 1H).

$^{13}C$ NMR ($CDCl_3$): 14.1, 20.6, 20.7, 20.9, 22.6, 25.2, 25.3, 29.1, 29.2, 29.3, 29.4, 29.5, 31.8, 70.0, 72.7, 98.2, 120.5, 121.2, 161.2, 164.0, 170.1 and 171.2.

MS exact mass calculated for $C_{21}H_{38}O_7N$ $(M+NH_4)^+$ 416.2648, found 416.2652.

## EXAMPLE 11

4-(1,2-Dihydroxyethyl)-2-triethylsilylfuran (prepared by the procedure of Example 2 using 5-triethylsilyl-3-furaldehyde in place of the corresponding trimethylsilyl furaldehyde and preparing the 5-triethylsilyl-3-furaldehyde by the alternative procedure using triethylsilyl chloride) is reacted with dodecanoyl chloride in the presence of triethylamine to give 4-(1-hydroxy-2-tridecanoyloxyethyl)-2-triethylsilylfuran. Treating this 1-hydroxy compound with acetic anhydride and pyridine gives 4-(1-acetoxy-2-tridecanoyl-oxyethyl)- 2-triethylsilylfuran.

A mixture of the above prepared furan and Rose Bengal in tetrahydrofuran is exposed to singlet oxygen by the procedure of Example 1 to give 4-(1-acetoxy-2-tridecanoyloxyethyl)-5-hydroxy-2(5H)-furanone.

## EXAMPLE 12

Reacting 4-(1,2-dihydroxyethyl)-2-triethyl- silylfuran with acetyl chloride and triethylamine give 4-(1-hydroxy-2-acetoxyethyl)-2-triethylsilylfuran. Reacting this intermediate with dodecanoyl chloride and triethylamine and oxidizing the resulting 4-(1-dodecanoyloxy-2-acetoxyethyl)-2-triethylsilylfuran gives 4-(1-dodecanoyloxy-2-acetoxyethyl)-5-hydroxy- 2(5H)-furanone.

## EXAMPLE 13

Using dodecyl isocyanate in place of dodecanoyl chloride in the procedure of Example 9 gives, as the product, 4-[1-acetoxy-2-(N-dodecylcarbamoyloxy) ethyl]-5-hydroxy-2(5H)-furanone.

## EXAMPLE 14

In the procedure of Example 11, using methyl methylphosphonochloridate in place of dodecanoyl chloride, the product is 4-[1-acetoxy-2-OP(O)($OCH_3$)($CH_3$) ethyl]-5-hydroxy-2(5H)-furanone.

## EXAMPLE 15

Reacting 4-[1-hydroxy-2-[3-(2-naphthyl)propoxy]ethyl]-2-trimethylsilfuran with tert-butyl lithium and phenylisocyanate gives 4-[1-phenylcarbamoyl-2-[3-(2-naphthyl)propoxy]ethyl]-2-trimethylsilylfuran. Oxidizing this intermediate with singlet oxygen gives 4-[1-phenylcarbamoyl-2-[3-(2-naphthyl)propoxy]ethyl]-5-hydroxy-2(5H)-furanone.

## EXAMPLE 16

As in Example 15, but substituting phenylisocyanate with ethyl chloroformate and carry through the reaction sequence gives 4-[1-ethoxycarbomyloxy-2-[3-(2-naphthyl)propoxy]ethyl]-5-hydroxy-2(5H)-furanone.

## EXAMPLE 17

As in Example 15, but substituting phenylisocyanate with chlorosulfonyl isocyanate and carry through the reaction sequence to give 4-[1-carbamoyl-2-[3-(2-naphthyl)propoxy]ethyl]-5-hydroxy-2(5H) furanone.

## EXAMPLE 18

As in Example 15, but substituting phenyl isocyanate with diethylchlorophosphate and carry through the reaction sequence to give 4-[1-diethylphosphonyl-2-[3-(2-naphthyl)propoxy]ethyl]-5-hydroxy-2(5H)-furanone.

EXAMPLE 19

Reacting 4-[1-diethylphosphonyl-2-[3-(2-naphthyl)propoxy]ethyl]-2-trimethylsilylfuran, obtained from Example 18, with bromotrimethylsilane and oxidizing this intermediate with singlet oxygen gives 4-[1-phosphonyl-2-[3-(2-naphthyl)propoxy]ethyl]-5-hydroxy-2(5H)-furanone.

EXAMPLE 20

The following test procedures may be used to demonstrate activity of the compound of this invention:

Inhibition of Phospholipase $A_2$

The effect of compounds of this invention on bee venom phospholipase $A_2$ is determined by the following procedure:

a. Bee venom phospholipasae $A_2$ in 10 µM HEPES (pH 7.4) with 1 mM $CaCl_2$ is incubated with vehicle or test agent for 1.0 hour at 41°.

b. 1.36 mM phosphotidylcholine, 2.76 mM Triton X-100 are dispersed in buffer by sonication and then mixed with L-3 phosphatidylcholine, 1-palmitoyl-2-(1-$^{14}$C) palmitoyl for 10 min.

c. Start the reaction by the addition of enzyme (0.495 units/ml).

d. Incubation for 15 sec. at 41°.

e. Reaction is terminated by addition of 2.5 ml of isopropanol: n-heptane: 0.5 M $H_2SO_4$ (40:10:1; v:v:v).

f. 2.0 ml n-heptane and 1.0 ml $H_2O$ added; mixture centrifuged.

g. 2.0 ml n-heptane removed and treated with 200-300 mg of silica gel HR60.

h. Samples centrifuged; 1 ml of n-heptane SN removed and added to 10 ml scintillation fluid.

i. Samples counted on a scintillation counter.

Inhibition of Phosphoinositide-specific Phospholipase C

The effect of compounds of this invention on phosphoinositide-specific phospholipase C may be determined by procedures described by Bennett et al, Molecular Pharmacology 32:587-593 (1987).

Mouse Ear Anti-Inflammatory Assay

Test compound and phorbol myristate acetate (PMA) are topically applied simultaneously to the pinnae of the left ears of mice. PMA alone is applied to the right ear. Three hours and 20 minutes after application, the mice are sacrificed, left and right ears removed, and standard sized bores taken. Edema (inflammation) is measured as the difference in weight between left and right ears [Van Arman, C.G., Clin Pharmacol Ther (1974) 16:900-904].

Inhibition of Ornithine Decarboxylase (ODC)

Tape-stripping mouse epidermis and TPA are quick and convenient methods of inducing ODC activity. M. Connor and N. Lowe (Cancer Res. 43, 5174, 1983; Brit. J. Dermatol. 275,.98, 1984) have studied the ability of retinoids to inhibit ODC. Trans-retinoic acid, 13-cis retinoic acid, and etretinate were all active at inhibiting ODC and therapeutically active in humans. Therefore, inhibition of ODC is an in vivo method to demonstrate the potential efficacy of drugs for epidermal hyperproliferation such as psoriasis. Lowe e. al. (J.

Amer. Acad. Dermatol. 6:697, 1982) have shown that polyamines and ODC are elevated in psoriasis.

In vitro methods have also been useful in determining the anti-hyperproliferative activity of drugs. C. Marcelo and J. Tomich (J. Invest. Dermatol. 81, 64s, 1983) have shown that neonatal mouse keratinocyte cultures can be used to identify drugs that inhibit DNA synthesis. More recently, R. Weiss, Eichner, R. and Sunn, T. T, J. Cell Biol., 98:1397-1406, (1984) have shown that epidermal cultures are in fact, a model of epidermal hyperproliferation and therefore a good model for testing drugs that inhibit hyperproliferation.

Calcium Channel (mobilization) inhibition assay.

Polymorphonuclear leukocytes (PMNa), gastric glands, $GH_3$ cells, A431 cells, spleen cells, human keratinocytes corneal cells, etc. were loaded with the $Ca^{2+}$ sensitive fluorescent dye, Fura-2. The appropriate cell type was chosen and the potency and efficacy of the anti-inflammatory furanones on calcium mobilization, calcium channel inhibition quantitated. The methods used for A431 cells listed below are representative of those used for other cells.

A431 cells were detached using a 5-10 min trypsin-EDTA treatment whereas $GH_3$ cells were treated 2 to 5 min with a 1% pancreatin solution. Cells were immediately washed twice in a 20 mM HEPES buffer (pH 7.4) containing 120mM NaCl, 6 mM KCl, 1 mM $MgSO_4$, 1 mg/ml glucose and 1 mg/ml pyruvate and 1.4mM calcium (medium A). Approximately 5 x $10^6$ cells wer suspended in medium A and incubated with 4µM fura-2-AM for 15 min at 37° C. After washing the fura-2 loaded cells, the uptake of dye was checked using fluorescence microscopy and found to be evenly distributed in the cytosol of all cells. Fluorescence was continuously recorded with a Perkin-Elmer LS-5 spectrofluorometer. The excitation wavelength was set at 340nm and emission wavelength set at 500nm. The cell suspension was continually stirred, maintained at 37° Cand equilibrated for approximately 5 min before addition of various agents. $[Ca^{2+}]_i$ was calculated using the following formula:

$$[Ca^{2+}]_i = 220 \frac{F-F_{min}}{F_{max}-F}$$

All fluorescence values were measured relative to a EGTA-quenched signal determined as follows: F was the relative fluorescence measurement of the sample. $F_{max}$ was determined by lysing the cells with digitonin (100µg/ml) in DMSO. After $F_{max}$ was determined the pH was adjusted to 8, with NaOH and $Ca^{2+}$ chelated with 3mM EGTA to totally quench the fura-2 signal and obtain $F_{min}$.

When quin-2 was used, cells were incubated with 10µM quin-2 at 37° C for 1 hour, washed and then used.

## Claims

1. A compound of the formula (I):

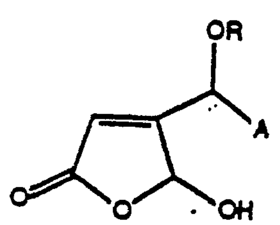

(I)

in which:

R is hydrogen, $C_1$-$C_{14}$ alkanoyl, $CONHR_3$ or $CO_2R_4$:

$R_3$ is H, phenyl or $C_1$-$C_4$alkyl;

$R_4$ is $C_1$-$C_6$ alkyl;

A is $CH_2O$-$R_1$ or

$CH(C_7-C_{14}$ alkyl); or
|
$OCOR_2$

when R is $C_1$-$C_{14}$ alkanoyl, A may be $CH_2OCOR_2$ or $CH_2OP(O)(OR_2)R_2$;
$R_1$ is $C_7$-$C_{14}$ alkanoyl,
N-($C_6$-$C_{14}$ alkyl) carbamoyl,
naphthyl-($C_1$-$C_6$ alkylene),
pyridyl-($C_1$-$C_6$ alkylene) or
methoxyethoxymethoxymethyl; and
$R_2$ is $C_1$-$C_4$ alkyl.

2. A compound of claim 1 in which R is $C_1$-$C_{14}$ alkanoyl and A is $CH_2O$-$R_1$.

3. A compound of claim 2 in which $R_1$ is $C_8$-$C_{14}$ alkanoyl, naphthylpropyl, pyridylpropyl or methoxyethoxymethoxymethyl.

4. A compound of claim 1 which is selected from:
4-[1-dodecanoyloxy-2-(2-methoxyethoxy)methoxyethyl]-5-hydroxy-2(5H)-furanone,
4-(1,2-didodecanoyloxyethyl)-5-hydroxy-2(5H)-furanone,
4-[1-acetoxy-2[3-(2-naphthyl)propoxy]ethyl]-5-hydroxy-2(5H)-furanone, and
4-[1-hydroxy-2-[3-(2-naphthyl)propoxy]ethyl]-5-hydroxy-2(5H)-furanone.

5. A pharmaceutical composition which comprises a pharmaceutical carrier and a therapeutically effective amount of a compound of any one of claims 1 to 4.

6. A pharmaceutical composition of claim 5 having an anti-inflammatory activity in mammals.

7. A compound of the formula (I) as defined in any one of claims 1 to 4, for use in the treatment of inflammation or an allergic response in a mammal.

8. A compound of the formula (I) as defined in any one of claims 1 to 4, for use in the treatment of psoriasis.

9. The use of a compound of the formula (I), as defined in any one of claims 1 to 4, in the preparation of a medicament for the treatment of inflammation or an allergic response in a mammal.

10. The use of a compound of the formula (I), as defined in any one of claims 1 to 4, in the preparation of a medicament for the treatment of psoriasis.

11. A process for the preparation of a compound of the formula (I) as defined in any one of claims 1 to 4, which process comprises the oxidation (for example with singlet oxygen in the presence of an initiator such as Rose Bengal) of a compound of the formula:

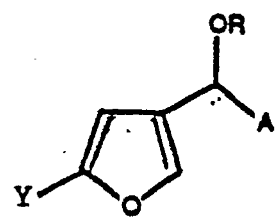

wherein R and A are as defined in claims 1 to 4 and Y is trimethylsilyl or triethylsilyl.

Claims for the following Contracting States: ES, GR

1. A process for the preparation of a compound of the formula (I):

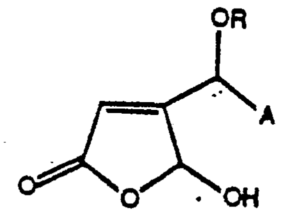

(I)

in which:

R is hydrogen, $C_1$-$C_{14}$ alkanoyl, $CONHR_3$ or $CO_2R_4$:

$R_3$ is H, phenyl or $C_1$-$C_4$alkyl;

$R_4$ is $C_1$-$C_6$ alkyl;

A is $CH_2O$-$R_1$ or

$CH(C_7$-$C_{14}$ alkyl); or
|
$OCOR_2$

when R is $C_1$-$C_{14}$ alkanoyl, A may be $CH_2OCOR_2$ or $CH_2OP(O)(OR_2)R_2$;

$R_1$ is $C_7$-$C_{14}$ alkanoyl,

N-($C_6$-$C_{14}$ alkyl) carbamoyl,

naphthyl-($C_1$-$C_6$ alkylene),

pyridyl-($C_1$-$C_6$ alkylene) or

methoxyethoxymethoxymethyl; and

$R_2$ is $C_1$-$C_4$ alkyl;

which process comprises the oxidation of a compound of the formula:

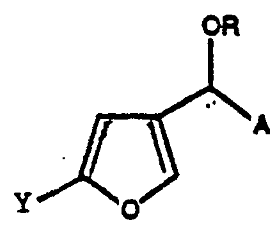

wherein R and A are as hereinbefore defined and and Y is trimethylsilyl or triethylsilyl.

2. A process of claim 1 in which R is $C_1$-$C_{14}$ alkanoyl and A is $CH_2O$-$R_1$.

3. A process of claim 2 in which $R_1$ is $C_8$-$C_{14}$ alkanoyl, naphthylpropyl, pyridylpropyl or methoxyethoxymethoxymethyl.

4. A process of claim 1 wherein the compound of the formula (I) is selected from;

4-[1-dodecanoyloxy-2-(2-methoxyethoxy)methoxyethyl]-5-hydroxy-2(5H)-furanone,

4-(1,2-didodecanoyloxyethyl)-5-hydroxy-2(5H)-furanone,

4-[1-acetoxy-2[3-(2-naphthyl)propoxy]ethyl]-5-hydroxy-2(5H)-furanone, and

4-[1-hydroxy-2-[3-(2-naphthyl)propoxy]ethyl]-5-hydroxy-2(5H)-furanone.

5. A process of any of claims 1 to 4 wherein the oxidation is effected using singlet oxygen in the presence of an initiator such as Rose Bengal.

6. A process for preparing a pharmaceutical composition containing a compound of the formula (I) as defined in any one of claims 1 to 4 and a pharmaceutically acceptable carrier, which process comprises bringing the compound into association with the pharmaceutically acceptable carrier.

7. A process according to claim 6 wherein the composition has anti-inflammatory activity in mammals.

8. The use of a compound of the formula (I), as defined in any one of claims 1 to 4, in the preparation of a medicament for the treatment of psoriasis.

9. The use of a compound of the formula (I), as defined in any one of the claims 1 to 4, in the

preparation of a medicament for the treatment of psoriasis.